# EUROPÄISCHE PATENTSCHRIFT

(11) **EP 2 395 952 B1**
(45) Veröffentlichungstag und Bekanntmachung des Hinweises auf die Patenterteilung: **16.04.2014**
(21) Anmeldenummer: 10807432.9
(22) Anmeldetag: 26.11.2010
(51) Int. Cl.: A61F 9/007, A61M 1/00

(54) **VORRICHTUNG ZUM ABSAUGEN VON FLÜSSIGKEIT AUS DEM MENSCHLICHEN ODER TIERISCHEN KÖRPER**
DEVICE FOR SUCTIONING LIQUID FROM A HUMAN OR ANIMAL BODY
DISPOSITIF POUR ASPIRER DU LIQUIDE CONTENU DANS LE CORPS HUMAIN OU ANIMAL

(30) Priorität: 26.11.2009 DE 102009055709
(43) Veröffentlichungstag der Anmeldung: 21.12.2011
(73) Patentinhaber: Geuder AG, 69126 Heidelberg (DE)
(72) Erfinder: GEUDER, Volker, 69126 Heidelberg (DE); BECHTEL, Helmut, 69121 Heidelberg (DE); FRAUENFELD, Dieter, 69121 Heidelberg (DE)
(74) Vertreter: Ullrich & Naumann
(86) Internationale Anmeldenummer: PCT/DE2010/001370
(87) Internationale Veröffentlichungsnummer: WO 2011/063794

(56) Entgegenhaltungen:
- EP-A1- 0 555 625
- EP-A1- 1 743 609
- EP-A2- 1 310 267
- US-A- 5 693 013
- US-A1- 2005 054 971

## Beschreibung

Die Erfindung betrifft eine Vorrichtung zum Absaugen von Flüssigkeit aus dem menschlichen oder tierischen Körper, insbesondere zur Aspiration von Flüssigkeit aus dem Auge während einer Augenoperation, mit einer Pumpe zum Erzeugen von Unterdruck in einer Unterdruckkammer, wobei die Pumpe über eine Unterdruckleitung oder dgl. mit der Unterdruckkammer strömungsverbunden ist und die Unterdruckkammer über eine Saugleitung mit einem chirurgischen Instrument strömungsverbindbar ist.

Gattungsbildende Vorrichtungen sind aus der Praxis bekannt und werden häufig als Aspirationsmodul bei Augenoperationen eingesetzt. Genauer gesagt dienen solche Module bzw. Vorrichtungen zum Absaugen von Flüssigkeit aus dem Auge, bspw. bei der Entfernung des pathologisch getrübten Linsenkörpers beim grauen Star. Zertrümmertes Linsenmaterial wird gemeinsam mit einer Spülflüssigkeit aus dem Augeninneren gesaugt, wobei über die Aspirationseinheit ein dosierter Unterdruck zum Absaugen mittels Handgerät generiert wird.

Bislang wurden solche Aspirationseinheiten mit Venturipumpen ausgestattet, die jedoch eine externe Pressluftversorgung benötigen. Dies ist aufwändig. Außerdem steht nicht überall dort, wo solche Operationen durchgeführt werden können, ein externer Pressluftanschluss zur Verfügung.

Auch ist bereits der Einsatz von Schlauchpumpen bei entsprechenden Aspirationsvorrichtungen bekannt, wobei es sich dabei um eine Verdrängerpumpe handelt. Solche Pumpen unterliegen aufgrund eines ständigen Walkens des Schlauchs einem ganz erheblichen Verschleiß und sind daher wartungsintensiv. Die Gefahr eines Schlauchschadens während des Betriebs, d. h. während einer Operation, ist erheblich.

Des Weiteren besteht bei entsprechenden Vorrichtungen, nämlich bei Aspirationseinheiten bzw. Aspirationsmodulen, der Bedarf, dass dem Operateur ein möglichst gleichmäßiger Unterdruck für sein Handgerät zur Verfügung gestellt wird. Er muss die Möglichkeit haben, je nach sich einstellender Operationssituation den Unterdruck möglichst stufenlos, zumindest aber in kleinsten Schritten, einzustellen. Ein pulsierender Unterdruck soll vermieden werden.

An dieser Stelle sei angemerkt, dass dem Operateur der im Handgerät erforderliche Unterdruck aus der Unterdruckkammer zur Verfügung gestellt wird, nämlich über die das chirurgische Instrument bzw. das Handgerät mit der Unterdruckkammer verbindende Saugleitung. In der Unterdruckkammer ist regelmäßig ein Feuchtigkeitsabscheider vorgesehen, um nämlich zu vermeiden, dass beim Absaugen kontaminierte Flüssigkeit bzw. Linsenmaterial zur Pumpe und in die Pumpe hinein gelangt.

Grundsätzlich ist es denkbar, eine Einstellung des Unterdrucks am Handgerät über eine äußerst große Unterdruckkammer zu generieren, wobei die Unterdruckkammer als Puffer für den Unterdruck dient. Zum Dosieren des Unterdrucks kann Luft aus der Atmosphäre beigemischt werden, so dass der Operateur, üblicherweise über einen Fußschalter, Vakuum aufbauen und abbauen kann, üblicherweise bei voller Pumpenleistung. Will man jedoch den Bauraum der gesamten Einheit reduzieren, sollte man auch die Unterdruckkammer so klein wie möglich bauen. Bei konventioneller Vorrichtung müsste die Pumpe ständig mit hoher Leistung laufen und müsste zur Reduzierung des Unterdrucks, je nach Bedarf, ständig Luft aus der Atmosphäre beigemengt werden. Die von der Pumpe ausgehenden Geräusche stören jedoch den Operateur erheblich, insbesondere dann, wenn die Pumpe stets mit hoher oder gar voller Leistung läuft.

Im Licht der voranstehenden Ausführungen liegt der Erfindung die Aufgabe zugrunde, eine Vorrichtung zum Absaugen von Flüssigkeiten aus dem menschlichen oder tierischen Köper derart auszugestalten und weiterzubilden, dass bei geringstmöglichen Betriebsgeräuschen eine Feineinstellung des erforderlichen Unterdrucks durch den Operateur möglich ist. Außerdem soll die Vorrichtung eine möglichst einfache Konstruktion aufweisen.

Die voranstehende Aufgabe ist durch die Merkmale des Patentanspruchs 1 gelöst. Danach ist die aus der EP 0555625 A1 bekannte gattungsbildende Vorrichtung dadurch gekennzeichnet, dass zur genauen Einstellung des in der Unterdruckkammer herrschenden Unterdrucks die Pumpe leistungsgeregelt und die Unterdruckleitung zwischen Pumpe und Unterdruckkammer geregelt belüftbar ist.

Zur genauen Einstellung des in der Unterdruckkammer herrschenden Unterdrucks ist einerseits die Pumpe leistungsregelt und andererseits die Unterdruckleitung zwischen Pumpe und Unterdruckkammer geregelt belüftbar.

Erfindungsgemäß sind zwei parallel zueinander angeordnete Pumpen vorgesehen, die asynchron arbeiten bzw. angesteuert sind. Durch diese Maßnahme ist die Bereitstellung eines konstanten Unterdrucks, unter Vermeidung einer pulsierenden Strömung, weitestgehend gewährleistet. Entsprechend einer solchen Anordnung fördern die beiden parallel angeordneten Pumpen wechselweise Strömungsmedium und generieren gemeinsam einen hinreichenden Unterdruck in der Unterdruckkammer. Außerdem hat sich herausgestellt, dass der parallele Betrieb zweier kleiner Pumpen im Vergleich zum Betrieb einer einzigen großen Pumpe die Geräuschemission ganz erheblich reduziert, und zwar bei Pumpen gleicher Bauart.

Erfindungsgemäß ist weiter erkannt worden, dass die Größe der Unterdruckkammer keineswegs zur genauen Einstellung des dem Operateur zur Verfügung gestellten Unterdrucks ausschlaggebend ist. Vielmehr wurde die Kombination zweier wesentlicher Merkmale realisiert, wonach nämlich die den Unterdruck generierende Pumpe leistungsgeregelt ist. Entsprechend dem Bedarf an Unterdruck fördert die Pumpe mehr oder weniger Fluidum. Gleichzeitig findet eine Belüftung der Unterdruckleitung zwischen Pumpe und Unterdruckkammer statt, nämlich in geregelter Weise. Der vom Operateur, bspw. über einen Fußschalter, angeforderte Unterdruck generiert sich somit aus der Leistungsregelung der Pumpe und der geregelten Belüftung der Unterdruckleitung, so dass eine genaue Einstellung des in der Unterdruckkammer herrschenden Unterdrucks mit einfachen Mitteln realisiert ist.

In ganz besonders vorteilhafter Weise ist die Pumpe als Membranpumpe, insbesondere als Diaphragmapumpe, ausgeführt. Dies hat den Vorteil, dass die beanspruchte Vorrichtung bzw. Aspirationseinheit insoweit autark arbeitet, als keine externe Druckluftversorgung erforderlich ist. Außerdem bauen solche Pumpen - Membranpumpe bzw. Diaphragmapumpe - äußerst klein und verursachen nur geringe Betriebgeräusche.

Wie bereits zuvor ausgeführt, wird in erfindungsgemäßer Weise nicht nur die Pumpe leistungsgeregelt, sondern findet auch eine geregelte Belüftung der Unterdruckleitung zwischen Pumpe und Unterdruckkammer statt. Dazu ist es von weiterem Vorteil, wenn die Belüftung der Unterdruckleitung über ein elektrisch betätigbares Belüftungsventil erfolgt, welches in der Unterdruckleitung oder in einer mit der Unterdruckleitung verbundenen Belüftungsleitung angeordnet ist. Bei dem Ventil handelt es sich in weiter vorteilhafter Weise um ein sog. Proportionalventil, welches stufenlos öffenbar und schließbar ist. Ein feines Hinzudosieren von Luft mit Atmosphärendruck ist somit zur Reduzierung des sich in der Unterdruckkammer einstellenden Unterdrucks möglich, wobei über die Pumpenleistung und die Regelung der Pumpenleistung eine dosierte Erhöhung des Unterdrucks möglich ist. In Bezug auf eine äußerst genaue Regelung, unter Nutzung einfacher Mittel, ist es von weiterem Vorteil, wenn das Belüftungsventil und die Pumpe bzw. Pumpen über einen gemeinsamen Regler ansteuerbar sind, wobei das Belüftungsventil umgekehrt proportional zur Pumpenleistung stufenlos öffnet und schließt. Dies bedeutet, dass das Belüftungsventil derart ausgelegt und geschaltet ist, dass es bei voller Pumpenleistung komplett geschlossen und bei minimaler Pumpenleistung nahezu offen bzw. bei abgeschalteter Pumpe komplett offen ist. Da sowohl das Ventil als auch die Pumpe stufenlos arbeiten, ist eine Feinstregulierung des Unterdrucks über beide Parameter möglich.

In weiter vorteilhafter Weise ist in die Unterdruckleitung zwischen Pumpe und Unterdruckkammer, vorzugsweise vor dem Belüftungsventil oder in der zum Belüftungsventil führenden Belüftungsleitung, ein weiteres Ventil vorgesehen, nämlich ein elektrisch betätigbares, vorzugsweise als Proportionalventil ausgeführtes Drosselventil geschaltet, welches - im Gegensatz zu dem zuvor erörterten Belüftungsventil - proportional zur Pumpenleistung stufenlos öffnet und schließt. Dies bedeutet, dass bei voller Leistung der Pumpe das Drosselventil komplett öffnet und mit abnehmender Pumpenleistung mehr und mehr schließt. Durch diese Maßnahme wird abermals einem pulsierenden Druck/Unterdruck entgegengewirkt, wobei das Ventil bei reduzierter Pumpenleistung einem Rückströmen und somit einer alternierenden Strömung in der Unterdruckleitung entgegen wirkt. Dies gilt insbesondere dann, wenn die Pumpe langsam bzw. mit geringer Förderleistung läuft.

An dieser Stelle sei angemerkt, dass gleich durch mehrere Maßnahmen einem Pulsieren in der Unterdruckleitung entgegengewirkt wird, nämlich durch das Zusammenwirken von geregelter Pumpe und geregeltem Belüftungsventil, durch die Vorkehrung des Drosselventils, welches bei geringer werdender Pumpenleistung zunehmend schließt und durch das Volumen innerhalb der Unterdruckkammer, die nach wie vor als Puffer wirkt. Außerdem sei weiter angemerkt, dass als Unterdruckschlauch ein Silikonschlauch verwendet wird, der eine zusätzliche Bedämpfung in Bezug auf das zu vermeidende Pulsieren im Strömungsmedium führt.

In weiter vorteilhafter Weise ist zwischen dem Belüftungsventil und der Unterdruckkammer, vorzugsweise in einer an die Unterdruckleitung angeschlossenen Druckmessleitung, ein Druckmesssensor zur Detektion des in der Unterdruckleitung herrschenden Unterdrucks vorgesehen. Das Messsignal des Drucksensors wird mit einem einen angeforderten Soll-Unterdruck präsentierenden, vorzugsweise analogen Vergleichssignal aus einem Microcontroller verglichen. Die Differenz wird über einen Differenzverstärker als Gleichspannungssignal über einen Pulsbreitenmodulator als pulsbreitenmoduliertes Signal über Leistungsendstufen der Pumpe bzw. den Pumpen und den Ventilen zu deren gemeinsamer Ansteuerung zugeführt. Dabei ist darauf zu achten, dass das als Belüftungsventil bezeichnete Proportionalventil umgekehrt proportional zur Pumpenleistung arbeitet. Das als Drosselventil bezeichnete Proportionalventil arbeitet proportional zur Pumpenleistung, drosselt somit bei geringer Pumpenleistung den Strömungspfad, wodurch einem Pulsieren des Strömungsmediums in ganz besonders raffinierter Weise entgegengewirkt wird.

Bei der erfindungsgemäßen Vorrichtung handelt es sich um eine eigenständige bauliche und funktionale Einheit, die für sich gesehen als Aspirationseinrichtung zu handhaben ist. Ebenso ist es denkbar, dass eine solche Vorrichtung als Modul einer Multifunktionseinheit für Augenoperationen dient, nämlich als eines von mehreren Modulen. Mit einer solchen Multifunktionseinheit könnte neben dem Unterdruck ein Strömungsmedium, Licht über eine Lichtleitfaser oder aber auch pulsierender Luftdruck zur Betätigung eines Cutters, etc. zur Verfügung gestellt werden.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines Ausführungsbeispiels der erfindungsgemäßen Vorrichtung anhand der Zeichnung zu verweisen. In Verbindung mit der Erläuterung des bevorzugten Ausführungsbeispiels der Vorrichtung anhand der Zeichnung werden auch im Allgemeinen bevorzugte Ausgestaltungen und Weiterbildungen der Lehre erläutert. In der Zeichnung zeigen
- Fig. 1: in einem schematischen Blockdiagramm die grundsätzliche Anordnung der einzelnen Bestandteile der erfindungsgemäßen Aspirationsvorrichtung und
- Fig. 2: in einem weiteren schematischen Blockdiagramm die Steuerung der Diaphragmapumpe und der Ventile der erfindungsgemäßen Vorrichtung.

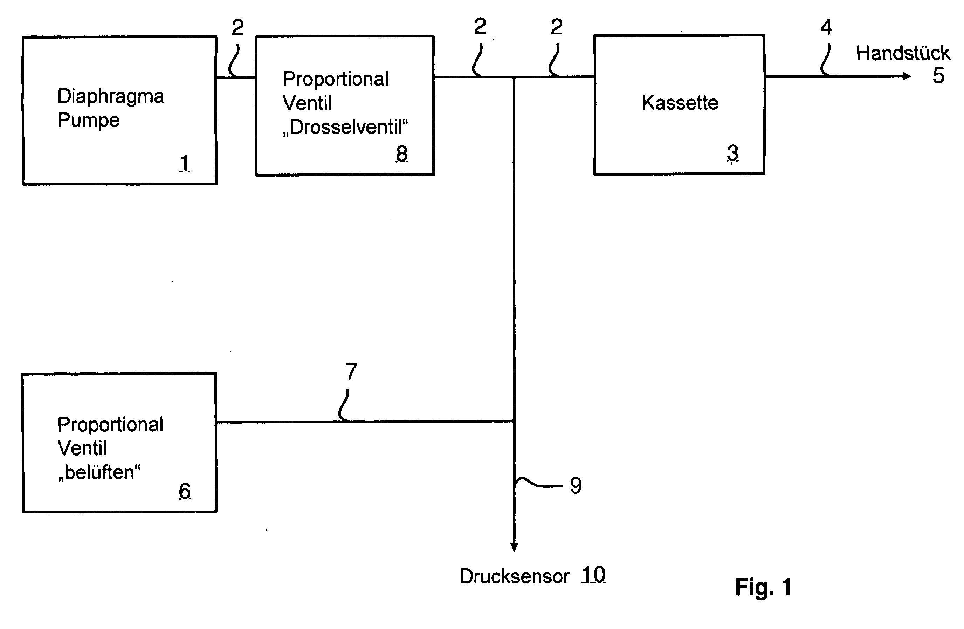

Fig. 1 zeigt in einem Blockdiagramm, schematisch, ein Ausführungsbeispiel einer erfindungsgemäßen Vorrichtung zum Absaugen von Flüssigkeit aus dem menschlichen oder tierischen Körper. Genauer gesagt handelt es sich dabei um eine Aspirationseinrichtung bzw. ein Aspirationsmodul.

Die in Fig. 1 gezeigte Vorrichtung umfasst eine Pumpe 1, die in erfindungsgemäßer Weise als Diaphragmapumpe ausgeführt ist.

Die Diaphragmapumpe 1 ist über eine Unterdruckleitung 2 mit einer Unterdruckkammer 3 in Form einer Kassette verbunden, so dass über die Diaphragmapumpe 1 in der Unterdruckkammer 3 ein Unterdruck erzeugt wird. Die Unterdruckkammer 3 ist wiederum über eine Saugleitung 4 mit einem Handstück 5 verbunden. Das Handstück 5 kann bspw. eine Sonde zum Einstecken bzw. Einführen in das menschliche Auge umfassen, über die Spülflüssigkeit und Linsentrümmer aus dem Auge in die Kassette bzw. Unterdruckkammer 3 gesaugt werden, bspw. im Rahmen einer Kataraktoperation.

Fig. 1 zeigt des Weiteren, dass zwischen der Diaphragmapumpe 1 und der Unterdruckkammer 3 ein elektrisch betätigbares Belüftungsventil 6 vorgesehen ist, nämlich zur Belüftung der Unterdruckleitung 2. Das Belüftungsventil 6 kann unmittelbar in der Unterdruckleitung 2 oder in einer mit der Unterdruckleitung 2 verbundenen Belüftungsleitung 7 angeordnet sein.

Bereits zuvor ist ausgeführt worden, dass das Belüftungsventil 6 als Proportionalventil ausgeführt ist und dabei stufenlos öffenbar und schließbar ist, wobei das Belüftungsventil 6 umgekehrt proportional zur Pumpenleistung stufenlos öffnet und schließt, nämlich derart, dass es bei voller Pumpenleistung komplett geschlossen und bei minimaler Pumpenleistung nahezu offen bzw. bei abgeschalteter Pumpe komplett offen ist.

Fig. 1 zeigt des Weiteren, dass in die Unterdruckleitung 2 zwischen der Diaphragmapumpe 1 und der Unterdruckkammer 3, vorzugsweise vor dem Belüftungsventil 6 oder in der zum Belüftungsventil 6 führenden Belüftungsleitung 7, ein elektrisch betätigbares, ebenfalls als Proportionalventil ausgeführtes Drosselventil 8 geschaltet ist, welches proportional zur Pumpenleistung stufenlos öffnet und schließt. Das Drosselventil 8 ist derart ausgelegt und gestaltet, dass es bei voller Leistung der Pumpe komplett öffnet und mit abnehmender Pumpenleistung schließt, um nämlich einem Pulsieren des Strömungsmediums entgegen zu wirken.

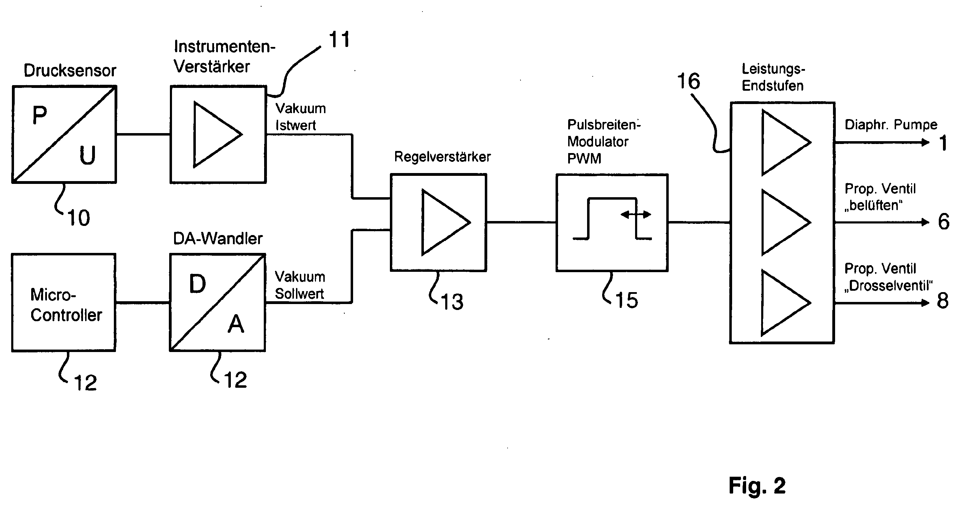

Fig. 1 lässt weiter erkennen, dass zwischen dem Belüftungsventil 6 und der Unterdruckkammer 3 in einer an die Unterdruckleitung 2 angeschlossenen Druckmessleitung 9 ein Druckmesssensor 10 zur Detektion des in der Unterdruckleitung 2 herrschenden Unterdrucks vorgesehen ist. Der Druckmesssensor 10 dient zur Regelung bei entsprechender Anforderung durch den Operateur. Die diesbezügliche Reglung ist im Blockschaltbild von Fig. 2 gezeigt.

Gemäß der Darstellung in Fig. 2 wird das Messsignal des Druckmesssensors 10 mit einem einen angeforderten Soll-Unterdruck repräsentierenden, analogen Vergleichssignal aus einem Microcontroller 11 verglichen. Das Vergleichssignal gelangt über einen DA-Wandler 12 als Vakuum-Sollwert zu einem Regelverstärker 13. Das vom Druckmesssensor 10 kommende Signal gelangt über einen Instrumentenverstärker 14 als Vakuum-Istwert zu dem Regelverstärker 13.

Das resultierende Signal wird über einen Pulsbreitenmodulator 15 Leistungsendstufen 16 zugeführt, von denen aus die Diaphragmapumpe 1, das Belüftungsventil 6 und das Drosselventil 8, über eine einzige Regeleinheit, angesteuert werden. Während das Belüftungsventil 6 umgekehrt proportional zur Diaphragmapumpe 1 arbeitet, arbeitet das Drosselventil 8 proportional dazu.

Hinsichtlich weiterer Merkmale, die sich den Figuren nicht entnehmen lassen, sei zur Vermeidung von Wiederholungen auf den allgemeinen Teil der Beschreibung verwiesen.

Schließlich sei angemerkt, dass das voranstehend erörterte Ausführungsbeispiel lediglich der beispielhaften Erläuterung der erfindungsgemäßen Vorrichtung dient, diese jedoch nicht auf das Ausführungsbeispiel einschränkt.

### Bezugszeichenliste

- 1: Pumpe, Diaphragmapumpe
- 2: Unterdruckleitung
- 3: Unterdruckkammer
- 4: Saugleitung
- 5: Handstück
- 6: Belüftungsventil
- 7: Belüftungsleitung
- 8: Drosselventil
- 9: Druckmessleitung
- 10: Druckmesssensor
- 11: Microcontroller
- 12: DA-Wandler
- 13: Regelverstärker
- 14: Instrumentenverstärker
- 15: Pulsbreitenmodulator
- 16: Leistungsendstufen

## Patentansprüche

1. Vorrichtung zum Absaugen von Flüssigkeit aus dem menschlichen oder tierischen Körper, insbesondere zur Aspiration von Flüssigkeit aus dem Auge während einer Augenoperation, mit einer Pumpe (1) zum Erzeugen von Unterdruck in einer Unterdruckkammer (3), wobei die Pumpe (1) über eine Unterdruckleitung (2) oder dgl. mit der Unterdruckkammer (3) strömungsverbunden ist und die Unterdruckkammer (3) über eine Saugleitung (4) mit einem chirurgischen Instrument strömungsverbindbar ist, wobei zur genauen Einstellung des in der Unterdruckkammer (3) herrschenden Unterdrucks die Pumpe (1) leistungsgeregelt und die Unterdruckleitung (2) zwischen Pumpe (1) und Unterdruckkammer (3) geregelt belüftbar ist **dadurch gekennzeichnet, dass** zwei parallel zueinander angeordnete, asynchron arbeitende Pumpen (1) vorgesehen sind.

2. Vorrichtung nach Anspruch 1, **dadurch gekennzeichnet, dass** die Pumpe (1) als Membranpumpe, insbesondere als Diaphragmapumpe (1), ausgeführt ist.

3. Vorrichtung nach Anspruch 1 oder 2, **dadurch gekennzeichnet, dass** die Belüftung der Unterdruckleitung (2) über ein elektrisch betätigbares Belüftungsventil (6) erfolgt, welches in der Unterdruckleitung (2) oder in einer mit der Unterdruckleitung (2) verbundenen Belüftungsleitung (7) angeordnet ist.

4. Vorrichtung nach Anspruch 3, **dadurch gekennzeichnet, dass** das Belüftungsventil (6) als Proportionalventil ausgeführt und dabei stufenlos öffenbar und schließbar ist.

5. Vorrichtung nach Anspruch 3 oder 4, **dadurch gekennzeichnet, dass** das Belüftungsventil (6) und die Pumpe(n) (1) über einen gemeinsamen Regler ansteuerbar sind, wobei das Belüftungsventil (6) umgekehrt proportional zur Pumpenleistung stufenlos öffnet und schließt.

6. Vorrichtung nach einem der Ansprüche 3 bis 5, **dadurch gekennzeichnet, dass** in die Unterdruckleitung (2) zwischen Pumpe (1) und Unterdruckkammer (3), vorzugsweise vor dem Belüftungsventil (6) oder in der zum Belüftungsventil (6) führenden Belüftungsleitung (7), ein elektrisch betätigbares, vorzugsweise als Proportionalventil ausgeführtes Drosselventil (8) geschaltet ist, welches proportional zur Pumpenleistung stufenlos öffnet und schließt.

7. Vorrichtung nach einem der Ansprüche 1 bis 6, **dadurch gekennzeichnet, dass** zwischen dem Belüftungsventil (6) und der Unterdruckkammer (3), vorzugsweise in einer an die Unterdruckleitung (2) angeschlossenen Druckmessleitung (9), ein Druckmesssensor (10) zur Detektion des in der Unterdruckleitung (2) herrschenden Unterdrucks vorgesehen ist.

8. Vorrichtung nach Anspruch 7, **dadurch gekennzeichnet, dass** das Messsignal des Druckmesssensors (10) mit einem einen angeforderten Soll-Unterdruck repräsentierenden, vorzugsweise analogen Vergleichssignal aus einem Microcontroller (11) verglichen und die Differenz über einen Differenzverstärker als Gleichspannungssignal über einen Pulsbreitenmodulator als pulsbreitenmoduliertes Signal über Leistungsendstufen der Pumpe bzw. den Pumpen (1) und den Ventilen (6, 8) zu deren gemeinsamer Ansteuerung zugeführt wird.

9. Multifunktionseinheit für Augenoperationen, **gekennzeichnet durch** eine Vorrichtung nach einem der Ansprüche 1 bis 8 als integrierte Aspirationseinheit.

## Claims

1. Device for removing liquid from the human or animal body by suction, in particular for aspirating liquid from the eye during an eye operation, having a pump (1) for generating vacuum in a vacuum chamber (3), wherein the pump (1) is in fluid communication with the vacuum chamber (3) *via* a vacuum line (2) or the like and the vacuum chamber (3) can be placed in fluid communication with a surgical instrument *via* a suction line (4), wherein, for the precise adjustment of the vacuum prevailing in the vacuum chamber (3), the pump (1) is output-controlled and the vacuum line (2) between the pump (1) and the vacuum chamber (3) can be ventilated in a controlled manner, **characterised in that** there are provided two pumps (1) which are arranged parallel to one another and work asynchronously.

2. Device according to claim 1, **characterised in that** the pump (1) is in the form of a membrane pump, in particular a diaphragm pump (1).

3. Device according to claim 1 or 2, **characterised in that** the ventilation of the vacuum line (2) is carried out *via* an electrically operable ventilation valve (6) which is arranged in the vacuum line (2) or in a ventilation line (7) connected to the vacuum line (2).

4. Device according to claim 3, **characterised in that** the ventilation valve (6) is in the form of a proportional valve and can be opened and closed in a continuously variable manner.

5. Device according to claim 3 or 4, **characterised in that** the ventilation valve (6) and the pump(s) (1) can be controlled *via* a common controller, wherein the ventilation valve (6) opens and closes in a continuously variable manner inversely proportionally to the pump output.

6. Device according to any one of claims 3 to 5, **characterised in that** there is connected in the vacuum line (2) between the pump (1) and the vacuum chamber (3), preferably upstream of the ventilation valve (6) or in the ventilation line (7) leading to the ventilation valve (6), an electrically operable throttle valve (8) which is preferably in the form of a proportional valve and which opens and closes in a continuously variable manner proportionally to the pump output.

7. Device according to any one of claims 1 to 6, **characterised in that**, between the ventilation valve (6) and the vacuum chamber (3), preferably in a pressure measurement line (9) connected to the vacuum line (2), there is provided a pressure measurement sensor (10) for detecting the vacuum prevailing in the vacuum line (2).

8. Device according to claim 7, **characterised in that** the measuring signal of the pressure measurement sensor (10) is compared with a preferably analog comparison signal from a microcontroller (11), which comparison signal represents a required desired vacuum, and the difference is fed *via* a differential amplifier as a d.c. voltage signal *via* a pulse width modulator as a pulse-width-modulated signal *via* power output stages to the pump or pumps (1) and the valves (6, 8) for the common control thereof.

9. Multifunctional unit for eye operations, **characterised by** a device according to any one of claims 1 to 8 as an integrated aspiration unit.

## Revendications

1. Dispositif permettant l'aspiration d'un liquide hors du corps humain ou d'un corps animal, plus particulièrement pour l'aspiration d'un liquide hors de l'oeil pendant une opération de l'oeil, avec une pompe (1) permettant de générer une dépression dans une chambre de dépression (3), la pompe (1) étant relié de manière fluidique par l'intermédiaire d'une conduite de dépression (2) ou autre avec la chambre de dépression (3) et la chambre de dépression (3) pouvant être reliée de manière fluidique avec un instrument chirurgical par l'intermédiaire d'une conduite d'aspiration (4), la pompe (1) étant régulée en puissance pour un réglage précis de la dépression régnant dans la chambre de dépression (3) et la conduite de dépression (2) entre la pompe (1) et la chambre de dépression (3) pouvant être ventilée de manière régulée, **caractérisé en ce que** deux pompes (1) fonctionnant de manière asynchrone sont prévues.

2. Dispositif selon la revendication 1, **caractérisé en ce que** la pompe (1) est conçue comme une pompe à membrane, plus particulièrement comme une pompe à diaphragme (1).

3. Dispositif selon la revendication 1 ou 2, **caractérisé en ce que** la ventilation de la conduite de dépression (2) a lieu à l'aide d'une soupape ventilation électrique (6) qui est disposée dans la conduite de dépression (2) ou dans une conduite de ventilation (7) reliée à la conduite de dépression (2).

4. Dispositif selon la revendication 3, **caractérisé en ce que** la soupape de ventilation (6) est conçue comme une soupape proportionnelle et peut donc être ouverte et fermée progressivement.

5. Dispositif selon la revendication 3 ou 4, **caractérisé en ce que** la soupape de ventilation (6) et la (les) pompe(s) (1) peut (peuvent) être contrôlée(s) à l'aide d'un régulateur commun, la soupape de ventilation (6) s'ouvrant et se fermant progressivement de manière inversement proportionnelle à la puissance de la pompe.

6. Dispositif selon l'une des revendications 3 à 5, **caractérisé en ce que**, dans la conduite de dépression (2) entre la pompe (1) et la chambre de dépression (3), de préférence avant la soupape de ventilation (6) ou dans la conduite de ventilation (7) conduisant à la soupape de ventilation (6), est branché une soupape d'étranglement électrique (8) conçue de préférence comme une soupape proportionnelle, qui s'ouvre et se ferme de manière proportionnelle à la puissance de la pompe.

7. Dispositif selon l'une des revendications 1 à 6, **caractérisé en ce que**, entre la soupape de ventilation (6) et la chambre de dépression (3), de préférence dans une conduite de mesure de pression (9) raccordée à la conduite de dépression (2), se trouve un capteur de mesure de pression (10) pour la détection de la dépression régnant dans la conduite de dépression (2).

8. Dispositif selon la revendication 7, **caractérisé en ce que** le signal de mesure du capteur de mesure de pression (10) est comparé à un signal de comparaison, de préférence analogique, représentant une dépression de consigne demandée, provenant d'un microcontrôleur (11) et al différence est introduite, par l'intermédiaire d'un amplificateur différentiel, en tant que signal de tension continue, par l'intermédiaire d'un modulateur de largeur d'impulsion, en tant que signal à largeur d'impulsion modulée, par l'intermédiaire des étages de puissance finaux dans la (les) pompe(s) (1) et les soupapes (6, 8), afin de permettre leur pilotage commun.

9. Unité multifonctionnelle pour opérations des yeux, **caractérisée par** un dispositif selon l'une des revendications 1 à 8 en tant qu'unité d'aspiration.
